# EUROPEAN PATENT SPECIFICATION

(11) **EP 1 430 034 B1**
(45) Date of publication and mention of the grant of the patent: **09.02.2005**
(21) Application number: 02767729.3
(22) Date of filing: 26.09.2002
(51) Int. Cl.: C07D 237/22, C07D 401/12, C07D 403/12, A61K 31/50, A61P 25/22, A61P 25/28

(54) **SUBSTITUTED ALKYLAMINOPYRIDAZINONE DERIVATIVES, PROCESS FOR THE PREPARATION THEREOF AND PHARMACEUTICAL COMPOSITION CONTAINING THE SAME**
SUBSTITUIERTE ALKYLAMINOPYRIDAZINONDERIVATE, VERFAHREN ZU IHRER HERSTELLUNG UND DIESE ENTHALTENDE PHARMAZEUTISCHE ZUBEREITUNGEN
DERIVES D'ALKYLAMINOPYRIDAZINONE SUBSTITUES, LEUR PROCEDE DE PREPARATION ET COMPOSITION PHARMACEUTIQUE LES CONTENANT

(30) Priority: 27.09.2001 HU 0103912
(43) Date of publication of application: 23.06.2004
(73) Proprietor: Egis Gyogyszergyar Rt., 1106 Budapest (HU); EGIS GYOGYSZERGYAR, Budapest X (HU)
(72) Inventor: BARKOCZY, Júzsef, H-1016 Budapest (HU); SIMIG, Gyula, H-1126 Budapest (HU); KOTAY NAGY, Péter, H-2600 Vác (HU); LEVAY, György, H-2092 Budakeszi (HU); GACSALYI, István, H-1201 Budapest (HU); MARTONNE MARKO, Bernadett, H-1171 Budapest (HU); SCHMIDT, Eva, H-1021 Budapest (HU); EGYED, András, H-1145 Budapest (HU); KOMPAGNE, Hajnalka, H-1221 Budapest (HU); LEVELEKI, Csilla, H-1029 Budapest (HU); MIKLOSNE KOVACS, Anikú, H-1141 Budapest (HU); SZENASI, Gábor, H-1031 Budapest (HU); WELLMANN, János, H-1026 Budapest (HU); HARSING, Lászlú Gábor, H-1071 Budapest (HU)
(74) Representative: Beszédes, Stephan G., Dr.
(86) International application number: PCT/HU2002/000097
(87) International publication number: WO 2003/027078

(56) References cited:
- EP-A- 0 372 305
- WO-A-92/12137
- PL-B- 164 079
- US-A- 3 931 176
- US-A- 5 395 934

## Description

### Field of the invention

The invention relates to substituted alkylaminopyridazinone derivatives, process for the preparation thereof and pharmaceutical composition containing the same.

### Background of the invention

Anxiety is a major CNS symptom accompanied by many psychiatric disorders, medical and surgical conditions and stress situations. Benzodiazepines such as diazepam, chlordiazepoxide, and alprazolam etc. are the most commonly used agents in the various anxiety disorders. However, sedative and amnestic side effects are a major disadvantage of these drugs especially in disorders affecting active, working populations. Moreover, withdrawal symptoms may occur following suspension of benzodiazepine administration after long term therapy. Therefore, finding of an effective anxiolytic/antistress compound without such undesirable side effects, low addictive potential and good safety features still remains one of the most challenging aims of CNS pharmacology research these days.

Piperazinylalkylamino-3(2H)-pyridazinone derivatives having blood pressure lowering effect and being suitable for the treatment of heart failure and peripheral circulatory disturbances are known from EP-A No. 372 305.

### Summary of the invention

The object of the invention is to provide new substituted alkylaminopyridazinone derivatives having useful pharmaceutical properties and free of disturbing side-effects.

The above object is achieved with the aid of the present invention.

According to the present invention there are provided new alkylaminopyridazinone derivatives of the general Formula 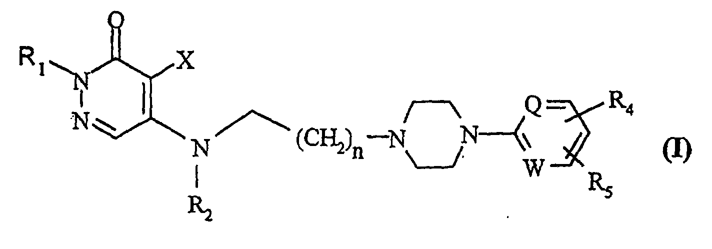 (wherein
R₁ is hydrogen or alkyl having 1-4 carbon atoms;
X is hydrogen or halogen;
R₂ is hydrogen or alkyl having 1-4 carbon atoms;
n is 1,2 or 3;
Q and W independently from each other stand for -N= or -CH=; and
R₄ and R₅ independently from each other represent hydrogen, halogen, trifluoromethyl or alkoxy having 1-4 carbon atoms) and pharmaceutically acceptable acid addition salts thereof.

### Description of the preferred embodiments

A preferable subgroup of the invention compounds of the general Formula I are those derivatives, in which
R₁ is hydrogen;
X is hydrogen;
n is 1;
Q and W independently from each other stand for -N= or -CH=; and
R₄ and R₅ independently from each other represent hydrogen, chlorine, fluorine, trifluoromethyl or methoxy
and pharmaceutically acceptable acid addition salts thereof.

The following compounds of the general Formula I possess particularly preferable pharmaceutical properties:
5-{2-[4-(methoxytrifluoromethylphenyl)-piperazine-1-yl]-ethylamino}-2*H*-pyridazine-3-one;
5-{2-[4-(2-fluorophenyl)piperazine-1-yl]ethyl-amino}-2*H*-pyridazine-3-one;
5-{2-[4-phenylpiperazine-1-yl]ethylamino}-2*H*-pyridazine-3-one;
5-[2-(4-pyridin-2-ylpiperazine-1-yl)ethylamino]-2*H*-pyridazine-3-one;
5-[2-(4-pyrimidin-2-ylpiperazine-1-yl)ethylamino]-2*H*-pyridazine-3-one;
5-{2-[4-(3-chlorophenyl)piperazine-1-yl]ethyl-amino}-2H-pyridazine-3-one;
5-{2-[4-(4-fluorophenyl)piperazine-1-yl]ethyl-amino}-2H-pyridazine-3-one
and pharmaceutically acceptable acid addition salts thereof.

The definition of the terms used throughout the body of the specification and the claims is as follows:
The term "halogen" means the fluorine, chlorine, bromine and iodine atoms, and is preferably chlorine.
The term "alkyl group having 1-4 carbon atoms" relates to straight or branched chained alkyl groups, e.g. methyl, ethyl, n-propyl, isopropyl, n-butyl, sec-butyl, isobutyl or tert-butyl etc.
The term "alkoxy" relates to straight or branched chained alkoxy groups, e.g. methoxy, ethoxy, isopropoxy or n-buthoxy, preferably methoxy.
The term "leaving group" relates to halogen (e.g. chlorine, bromine) or alkylsulfonyloxy groups (e.g. methylsulfonyloxy) or arylsulfonyloxy groups (e.g. benzylsulfonyloxy, p-toluene-sulfonyloxy).
The term "pharmaceutically suitable acid addition salt" of the substituted alkylaminopyridazinone derivatives of the general Formula I, relates to non-toxic acid addition salts of the compounds formed with inorganic acids such as hydrochloric acid, hydrobromic acid, sulfuric acid, phosphoric acid etc. or organic acids such as formic acid, acetic acid, maleic acid, fumaric acid, lactic acid, tartaric acid, succinic acid, citric acid, benzenesulfonic acid, p-toluenesulfonic acid, methanesulfonic acid etc.

According to a further feature of the present invention there is provided a process for the preparation of compounds of the general Formula I
(wherein
R₁ is hydrogen or alkyl having 1-4 carbon atoms;
X is hydrogen or halogen;
R₂ is hydrogen or alkyl having 1-4 carbon atoms;
n is 1, 2 or 3;
Q and W independently from each other stand for -N= or -CH=; and
R₄ and R₅ independently from each other represent hydrogen, halogen, trifluoromethyl or alkoxy having 1-4 carbon atoms) and pharmaceutically acceptable acid addition salts thereof which comprises
a) reacting a compound of the general Formula 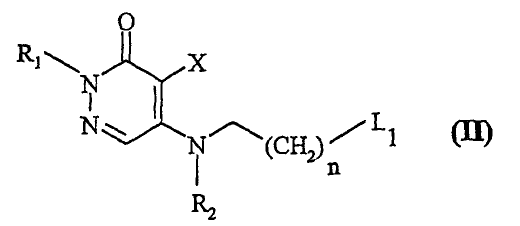 (wherein
   L₁ is leaving group and R₁, R₂, X and n are as stated above) with an amine of the general Formula 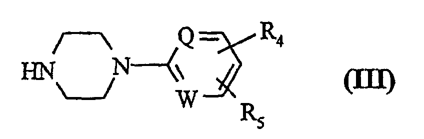 (wherein Q, W, R₄ and R₅ are as stated above); or
b) reacting a compound of the general Formula 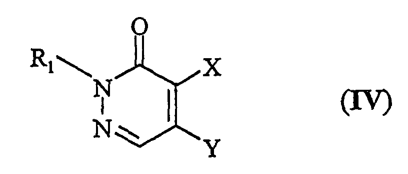 (wherein Y is halogen and R₁ and X are as stated above) with a compound of the general Formula 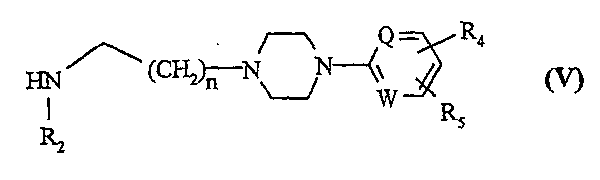 (wherein R₂, n, Q, W, R₄ and R₅ are as stated above); and, if desired, subjecting a compound of the general Formula I, wherein X stands for halogen, to catalytic dehalogenation to obtain a compound of the general Formula I or its hydrochloride salt, wherein X is hydrogen; and/or
   converting a compound of the general Formula thus obtained into a pharmaceutically acceptable acid addition salt thereof or setting free a compound of the general Formula I from an acid addition salt.

In case of processes (a) and (b) of the invention, the reactions are carried out in a manner similar to the known analogous processes, see e.g. March, J.: Advanced Organic Chemistry, Reactions, Mechanism and Structure, 4^{th} Edition, John Wiley and Sons, New York, 1992.

When a substituted alkylaminopyridazinone derivative of the Formula I, wherein X stands for halogen, preferably chlorine, is subjected to catalytic hydrogenation, then dehalogenation proceeds and the corresponding substituted alkylaminopyridazinone derivative of the Formula I or its hydrochloride salt, wherein X stands for a hydrogen atom is formed.

The catalytic hydrogenation is carried out in an analogous manner as the processes described in the literature [e.g. March, J.: Advanced Organic Chemistry, Reactions, Mechanism and Structure, 4^{th} Edition, John Wiley and Sons, New York, 1992]. As the hydrogen source, for example, hydrogen gas, hydrazine, hydrazine hydrate, formic acid, a trialkylammonium formate or an alkali metal formate can be used. The catalyst is suitably palladium, platinum oxide, Raney nickel etc. The reaction can be performed in the presence or absence of an acid binding agent. As the acid binding agent, an inorganic base such as sodium hydroxide or an organic base such as hydrazine, triethyl amine, diisopropyl ethyl amine etc. can be used. The reaction can be carried out in an indifferent protic or aprotic solvent or a mixture thereof. The protic solvent is, for example, an alkanol, water or mixtures thereof, the aprotic solvent is suitably dioxane, tetrahydrofurane or dichloromethane. In general, the reaction temperature is 0-150°C, preferably 20-100°C.

The preparation of the acid addition salt from the free base of the general Formula I and the liberation of the base from the acid addition salt are carried out in a manner known *per se*.

The alkylaminopyridazinone derivatives of the general Formula II used as the starting compounds can be prepared by the process described in the International Patent Application No. PCT/HU98/00054; (WO 99/64402).

The amines of the general Formula III are partly known compounds. The novel ones can be prepared in an analogous way [Pollard et al., J.Am.Chem.Soc., 56, 2199 (1934)].

The dihalopyridazinone derivatives of the general Formula IV are partly known. The novel compounds can be prepared by using the methods known from the literature [Homer et al., J. Chem. Soc., 1948, 2194].

The compounds of the general Formula V can be prepared from the corresponding amine of the general Formula III in a manner known *per se* [Shigenaga, S. et al., Arch. Pharm., 329(1), 3-10 (1996); Janssens, F. et al., J. Med. Chem., 28(12), 1934-1943 (1985); He Xiao Shu et al., Bioorg. Med. Chem. Lett., 7(18), 2399-2402 (1997)].

The pharmacological effect of the substituted alkylaminopyridazinone derivatives of the general Formula I was studied in the following test.

### Methods and Results

### Effects on blood pressure

The experiments were performed in conscious, freely moving, male Wistar rats using a radiotelemetry system (Data Sciences International, St. Paul, Minnesota, USA). Prior to treatments rats were implanted with transmitters (type: TL11M2-C50-PXT) that permitted continuos monitoring of arterial blood pressure. Under sterile surgical conditions, catheter of the transmitter was introduced into the abdominal aorta for measurement of arterial blood pressure and the transmitter was sutured to the abdominal wall of animals anaesthetised with pentobarbital-Na (60 mg/kg, i.p.; Nembutal inj. Phylaxia-Sanofi, Budapest). After surgery the animals were treated with an antibiotic (1 ml/kg i.m. Tardomyocel comp. inj. ad us. vet., Bayer AG, Leverkusen, Germany). A 7 day postoperative recovery period was allowed. Radio signals emitted by the transmitters were detected by RLA1000 type receivers placed under each animal's cage. The data were collected, saved and evaluated using the Dataquest IV. software from Data Sciences. The computer was set to sample the parameters for 10 seconds in every second minute.
The test substances or vehicle (methyl cellulose 0.4% w/v) were administered orally by gavage in a volume of 1 ml/kg at about 10 a.m. The effects of test items were measured for 6 hours. The effect of each compound was compared with that caused by vehicle treatment using two-way analysis of variance for repeated measures with Scheffe's post hoc test.

Data obtained are shown in the Table 1. None of the compounds examined reduced blood pressure of the test animals.

**Table 1**

| Effects of different test items or vehicle on mean arterial blood pressure for 6 hours after treatment in conscious rats | | | | | |
|---|---|---|---|---|---|
| **Example** | **Mean blood pressure** | | | | **Results of statistical evaluation** |
| | after treatment with placebo (mmHg) | | after treatment with test compound (mmHg) | | |
| | Mean | S.E. | Mean | S.E. | |
| 7 | 92.6 | 3.3 | 92.7 | 3.2 | N.S. |
| S.E. = Standard Error (of the mean); N.S. = Not significant statistically (when compared to placebo) | | | | | |

According to data presented here the compounds of present invention have no effect on blood pressure, indicating the lack of antihypertensive potential.

### Anxiolytic effect

### Vogel lick-conflict

Experiments were performed in a PC operated system (LIIKOSYS, Experimetria, Hungary) consisted of 8 test chambers (20 cm x 20 cm x 20 cm Plexiglas boxes) each of which was equipped with a water fountain system mounted at appropriate height on the wall of the chamber and metal grid floor for delivering electric shocks. 160-180 g male Wistar rats (N=8) were deprived of drinking water 48 h and fasted for 24 h prior to test. Test and reference compounds or vehicle were administered *intraperitoneally,* 30 min prior to test. All procedures were carried out in a quiet, air-conditioned room between 07:30 and 13:00 h at an ambient temperature of 23 ±2 °C. At the beginning of the test the animals were placed in the test chamber where they had free access to drinking water for a 30 s grace period. After that, electric shocks (600 µA, 0.6 s) were applied through the drinking spout following every 20 licks during the 5 min test period (Vogel et al, 1971). Number of punished licks were recorded and stored by an IBM compatible computer. Means ± SEM of numbers of tolerated shocks were calculated in each group, statistical analysis of data was performed by one way ANOVA followed by Duncan's test (STATISTICA). The results obtained are shown in Table 2. Diazepam [7-chloro-1,3-dihydro-1-methyl-5-phenyl-2H-1,4-benzodiazepine-2-one] was used as the reference substance.

**Table 2**

| Vogel's drinking conflict test | |
|---|---|
| **Compound (Example No.)** | **MED in mg/kg ip.** |
| 1 | 20.0 |
| 2 | 20.0 |
| 3 | 5.0 |
| 4 | 10.0 |
| 5 | 20.0 |
| 7 | 20.0 |
| Diazepam | 5.0 |

The data of Table 2 indicate that the substituted alkylaminopyridazinone derivatives of the general Formula I have significant anxiolytic effect equivalent to that of diazepam.

### Elevated plus-maze test in rats

Tests have been performed as described by Pellow and co-workers [J. Neurosci. Methods, 14, 149 (1985)]. A wooden cross, 15 cm wide with 100 cm long arms was used for the experiments. The sides and ends of two opposite arms of the cross were equipped with 40 cm high walls, however, the arms were open to the 15x15 cm central area (closed arms). The two other opposite arms were not encircled by walls (open arms).

Male Sprague-Dawley rats weighing 200-220 g were used for the experiments. The animals were placed in the central area of the equipment 60 min after treatment and the following four parameters have been observed for the 5 min test time:
- time spent in the open arms,
- time spent in the closed arms,
- number of entries into the open arms,
- number of entries into the closed arms.

The effect was expressed as percent increase in either the time (measured in sec) spent in the open arms or number of entries into the open arms. MEDs (minimal effective dose) were determined for each compound regarding the time spent in the open arms.
The results obtained are summarized in Table 3. Buspirone [8-{4-[4-(2-pyrimidinyl)-1-piperazinyl]butyl}-8-azaspiro[4,5]decane-7,9-dione] was used as the reference substance.

**Table 3**

| Elevated plus-maze test in rats | |
|---|---|
| **Compound (Example No.)** | **MED in mg/kg po.** |
| 3 | 0.1 |
| Buspirone | 3.0 |

From Table 3 it is evident that the substituted alkylaminopyridazinone derivatives of the general Formula I have outstanding anxiolytic activity in the above test, considerably exceeding the efficacy of the reference substance.

### Sedative effect

### Inhibition of spontaneous motor activity

The effect on spontaneous motor activity was investigated according to Borsy and co-workers [Borsy, J. et al, Arch. Int. Pharmacodyn., 124, 180-190 (1960)] in a ten channel Dews instrument, with 1-1 animal in each channel. Animals were placed into the instrument 60 min after per os treatment with either vehicle or test compound, and interruptions of infrared beams were recorded for 30 min. From these data, 50 per cent inhibitory doses (ID₅₀)have been determined by regression analysis. The results obtained are shown in Table 4. Diazepam was used as the reference substance.

**Table 4**

| Inhibition of spontaneous motility in mouse | |
|---|---|
| **Compound (Example No.)** | **ID**_{**50**} **in mg/kg po.** |
| 2 | 21.0 |
| 3 | 60.0 |
| 5 | >100.0 |
| Diazepam | 7.0 |

In contrast to the diazepam used as the reference substance, the tested substituted alkylaminopyridazinone derivatives of the general Formula I display sedative effect only in a relatively high dose.

Based on the above test data, the substituted alkylaminopyridazinone derivatives of the general Formula I are effective in the treatment of various clinical patterns connected with anxiety. In case of certain compounds, the anxiolytic potential exceeds by several orders of magnitude the effect of the marketed reference substances (diazepam, buspirone). Sedative side-effect appears only in a dose that is multiple of the one needed to produce the expected therapeutical effect. This means that the substituted alkylaminopyridazinone derivatives of the general Formula I do not have sedative, life quality deteriorating side-effects which are characteristic of benzodiazepines.

### Effect of cognition and memory

Male Wistar rats weighing 200-220g were used. The animals were obtained from Charles River Co. They were kept in a room with normal 12-12 h light-dark cycle (light on: 06:00) at relative humidity of 60±10 %.

The experiment was performed in a five-channel "step through"-type passive avoidance learning apparatus. The equipment consisted of two adjacent Plexi-glass boxes of 20x20x16 cm. One of them was made of regular transparent Plexi-glass and the other one was made of black, non-transparent Plexi-glass. The boxes were connected with a 7.5x8 cm passage way, equipped with a computer-controlled guillotine-door. The passage of the rats through the door was detected by infrared photocells arranged in two parallel lines in the opening of the passage way. The door was automatically closed when the animals passed through. The dark compartment was equipped with stainless steel grid floor through which electric foot shocks could have been delivered to the animals. A 10 W light bulb was installed above the passage way in the light compartment.

The experiment was performed on two consecutive days, in two sessions which were 24 h apart from each other.

On Day 1 (Acquisition) the animals acquired information about the situation (grid floor shock in the dark compartment), on Day 2 (Retention) they recalled the acquired information to avoid punishment ("if I go into the dark I will be punished, so I stay outside in the light").

### Day 1 (Acquisition)

The individually numbered animals were placed into the light compartment of the equipment. After 30 s the guillotine door was opened and the rats could freely pass to the dark (considered as safe) compartment. Step through latency was automatically determined. (Step-trough latency is the time period spanning from door opening to the time when the animal passed into the dark compartment.) The door was closed then, and the timer was automatically stopped. An electric foot shock of 1.2 mA lasting 2.5 s was applied to the animal through the grid floor 3 s after the door has been closed, except for rats in the absolute control group (no shock + vehicle treated). Test animals were removed from the dark compartment immediately after foot shock has been delivered. The function of the absolute control group was to show that shocked animals will remember the unpleasant foot shock as revealed by increased latency time when compared to absolute control. That is the essence of acquisition.

### Day 2 (Retention)

After 24 h, the animals were placed again in the light compartment of the test apparatus and step-through latency was measured as described at Acquisition day, except that no foot shock was applied to the animals in any group on the second day. A maximum of 180 s time interval was available for the rats to pass into the dark compartment. The animals were removed from the light compartment if they did not pass to the dark compartment within the 180 s test period.

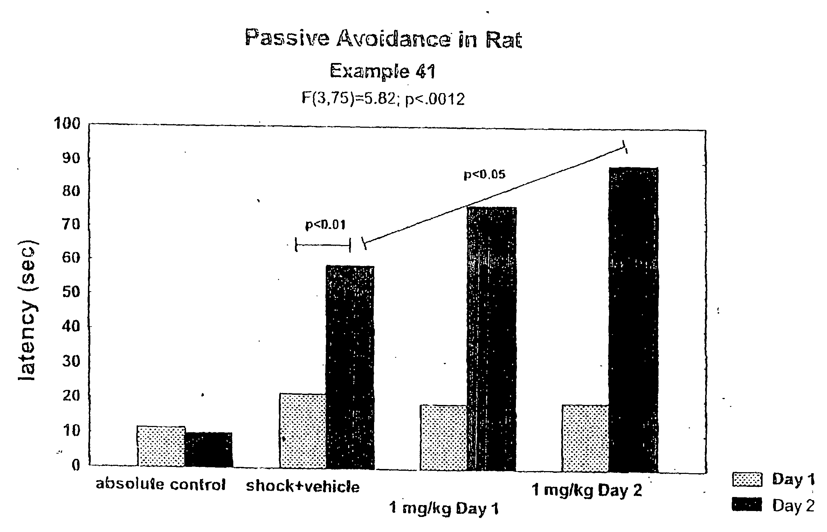

The investigators surprisingly found that the invention compounds significantly increased step-through latency into the dark compartment of the passive avoidance apparatus after Day 2 administration of the compound (**Fig 1**).
It is shown on Fig 1 that in absolute control group(no shock, untreated), step-trough latency was approximately the same on both experimental days (meaning that there was nothing to recall and avoid on the second day).

In the shocked, vehicle-treated control group the unavoidable 1.2 mA foot shock resulted in a significantly increased step-through latency on Day 2 when compared to absolute control. The experimental animals recalled the annoying experience (foot shock) in the dark, therefore, they pass into the dark compartment with a significantly longer time (increased latency).
In the treated groups this augmented latency has been further increased after the Day 2 treatment indicating that the retention of memory has been improved.
These surprising effects are not evident since anxiolytic compounds (i.e. diazepam) have a deleterious effect on memory.
From therapeutic point of view the advantageous effect of compounds of general Formula I on learning and memory signifies that the compounds could be appropriate for treating and/or preventing diseases or conditions accompanying diseases wherein learning or memory functions are suffering a loss or there is a possibility to suffering a loss. Such diseases are, but not limited to - as mentioned earlier - Alzheimer's disease, Korsakoff syndrome, Huntington's disease, Parkinson's disease and mental decline due to ageing processes, impairment of the cognitive functions or to exposure to toxic substances as well.

### Summary

The compounds of general Formula I possess surprisingly considerable anxiolytic properties without sedative side effects in their anxiolytic dose range. In addition to the anxiolytic efficacy, the compounds of general Formula I have advantageous effects on cognition and memory. According to our studies the compounds of general Formula I surprisingly have no antihypertensive potential.

The compounds of the invention and pharmaceutically suitable acid addition salts thereof can be used as active ingredients in pharmaceutical compositions.

Furthermore, the invention relates to a pharmaceutical composition comprising a substituted alkylaminopyridazinone derivatives of the general Formula I or a pharmaceutically suitable acid addition salt thereof and one or more conventional carriers.

The pharmaceutical composition of the invention contains, in general, 0.1 to 95 per cent by weight, preferably 1 to 50 per cent by weight, suitably 5 to 30 per cent by weight of the active ingredient.

The pharmaceutical composition of the invention is suitable for peroral, parenteral, rectal or transdermal administration or for local treatment, and can be solid or liquid.

The solid pharmaceutical compositions suitable for peroral administration may be powders, capsules, tablets, film-coated tablets, microcapsules etc., and can comprise binding agents such as gelatine, sorbitol, poly(vinyl-pyrrolidone) etc.; filling agents such as lactose, glucose, starch, calcium phosphate etc.; auxiliary substances for tabletting such as magnesium stearate, talc, poly(ethylene glycol), silica etc.; wetting agents such as sodium laurylsulfate etc. as the carrier.

The liquid pharmaceutical compositions suitable for peroral administration may be solutions, suspensions or emulsions and can comprise e.g. suspending agents such as gelatine, carboxymethylcellulose etc.; emulsifiers such as sorbitane monooleate etc.; solvents such as water, oils, glycerol, propylene glycol, ethanol etc.; preservatives such as methyl p-hydroxybenzoate etc. as the carrier.

Pharmaceutical compositions suitable for parenteral administration consist of sterile solutions of the active ingredient, in general.

Dosage forms listed above as well as other dosage forms are known *per se*, see e.g. Remington's Pharmaceutical Sciences, 18^{th} Edition, Mack Publishing Co., Easton, USA (1990).

The pharmaceutical composition contains dosage unit, in general. A typical dose for adult patients amounts to 0.1 to 1000 mg of the compound of the general Formula I or a pharmaceutically suitable acid addition salt thereof calculated for 1 kg body weight, daily. The daily dose can be administered in one or more portions. The actual dosage depends on many factors and is determined by the doctor.

The pharmaceutical composition is prepared by admixing a compound of the general Formula I or a pharmaceutically suitable acid addition salt thereof to one or more carrier(s), and converting the mixture obtained to a pharmaceutical composition in a manner known *per se*, Useful methods are known from the literature, e.g. Remington's Pharmaceutical Sciences mentioned above.

According to a further feature of the present invention there is provided the use of compounds of the general Formula I or a pharmaceutically acceptable acid addition salt thereof as pharmaceutical active ingredient.

According to a preferred embodiment of the above feature of the invention there is provided the use of compounds of the general Formula I or pharmaceutically acceptable acid addition salts thereof as anxiolytic and cognition enhancing pharmaceutically active ingredients.

According to a still further feature of the present invention there is provided a method of anxiolytic and cognition enhancing treatment of anxiolytic conditions which comprises administering to the person in need of such treatment a pharmaceutically effective amount of a compound of the general Formula I according to claim 1 or a pharmaceutically acceptable acid addition salt thereof.

Further details of the present invention are to be found in the following Examples without limiting the scope of protection to said Examples.

### EXAMPLES

### Example 1

### Preparation of 5-{2-[4-(methoxytrifluoromethylphenyl)-piperazine-1-yl]-ethylamino}-2H-pyridazine-3-one trihydrochloride

3.7 g (0.0086 moles) of 4-chloro-5-{2-[4-(methoxytrifluoromethyl-phenyl)piperazine-1-yl]ethylamino}-*H*-pyridazine-3-one, 370 cm³ of methanol, 3.2 cm³ (0,018 moles) of diisopropyl-ethylamine and 3.7 g of palladium on carbon catalyst consisting of 8% of Pd, 28% of C and 64% of H₂O are transferred into an autoclave. The reaction mixture is stirred at room temperature and under a hydrogen pressure of 10 atm for 4 hours. Then, the excess hydrogen is let out from the autoclave, the reaction mixture is heated to reflux temperature and stirred at this temperature for 5 minutes, then filtered while hot, and the catalyst is washed three times using 33 cm³ of a 1:1 mixture of methanol and dichloromethane each time. The combined filtrates are evaporated under reduced pressure, and the residue is subjected to chromatography over a silica gel column using a 19:1 mixture of chloroform and methanol as the eluent. The fractions containing the product are evaporated, the residue is dissolved in a mixture of ethyl acetate and diethyl ether, and, to the solution obtained, ether containing hydrogen chloride are added, drop by drop. The crystals that precipitate are stirred for half an hour under cooling with ice water, then filtered and washed with diethyl ether. The product is dried at 80 °C over phosphorus pentoxide *in vacuo* for 3 hours.

Thus, 1.84 g (54 %) of the title compound are obtained. M.p.: 238-240 °C

| Analysis: for C₁₈H₂₅Cl₃F₃N₅O₂ (506.79) | | | | |
|---|---|---|---|---|
| calculated | C 42.66 %, | H 4.97 %, | N 13.82 %, | Cl 20.99 %; |
| found | C 42.53 %, | H 5.01 %, | N 13.63 %, | Cl 20.69 %. |

IR (KBr): 3294, 2340, 1630, 1330, 1115;
¹H-NMR (DMSO-d₆, i400): 13.23 (b, 1H), 11.49 (b, 1H), 8.43 (b, 1H), 7.90 (bs, 1H), 7.40 (d, J=8.5 Hz, 1H), 7.18 (d, J=8.7 Hz, 1H), 7.15 (s, 1H), 6.05 (bs, 1H), 3.89 (s, 3H), 3.13-3.75 (m, 12H).
¹³C-NMR (DMSO-d₆, i400): 162.14, 154.81, 150.30, 139.98, 134.04, 124.68 (q, J=271.6 Hz), 121.51 (q, J=31.7 Hz), 120.92 (q), 114.81 (q), 112.22, 93.60, 56.13, 53.09, 51.30, 46.69, 36.49.

### Example 2

### Preparation of 5-{2-[4-(2-fluorophenyl)piperazine-1-yl]ethylamino}-2H-pyridazine-3-one

2.5 g (0.0071 moles) of 4-chloro-5-{2-[4-(2-fluorophenyl)piperazine-1-yl]ethylamino}-2*H*-pyridazine-3-one, 400 cm³ of a 9:1 mixture of methanol and distilled water and 2.5 g of palladium on carbon catalyst consisting of 8% of Pd, 28% of C and 64% of H₂O are weighed into an apparatus of 1000 cm³ volume equipped with a reflux condenser connected to a bubbling device. 1.4 cm³ of hydrazine hydrate are added, drop by drop, to the reaction mixture that is then stirred at reflux temperature for 1 hour. The mixture is filtered while hot, and the catalyst is washed three times using 33 cm³ of a 1:1 mixture of methanol and dichloromethane. The combined filtrates are evaporated, and the residue is dissolved in 25 cm³ of a 8:1 mixture of ethanol and water under heating, the solution is filtered, and the filtrate is evaporated to the fifth of the original volume. After cooling, the crystals separated are stirred for further half an hour under cooling with ice water, then filtered and washed with diethyl ether. The product is dried at 40°C over phosphorus pentoxide *in vacuo* for 3 hours.

Thus, 1.91 g (84..8 %) of the title compound are obtained. M.p.: 103-105 °C.

| Analysis: for C₁₆H₂₀FN₅O (317.37) | | | |
|---|---|---|---|
| calculated | C 60.55 %, | H 6.35 %, | N 22.07 %; |
| found | C 60.25 %, | H 6.34 %, | N 21.89 %. |

IR (KBr): 3272, 3122, 1633, 1550.
¹H-NMR (DMSO-d₆, i400): 11.87 (bs, 1H), 8.35 (d, J=4.8 Hz, 2H), 7.48 (d, J=2.6 Hz, 1H), 6.85 (bt, J=5.1 Hz, 1H), 6.62 (t, J=4.7 Hz, 1H), 5.40 (∼s, 1H), 3.73 (m, 4H), 3.14 (∼q, J=6.0 Hz, 2H), 2.50 (m)
¹³C-NMR (DMSO-d₆, i400): 162.42, 161.38, 158.08, 149.44, 131.73, 110.28, 94.38, 55.85, 52.71, 43.40, 39.16.

### Example 3

### Preparation of 5-{2-[4-phenylpiperazine-1-yl]ethylamino}-2H-pyridazine-3-one

3.3 g (0.01 moles) of 4-chloro-5-[2-(4-phenylpiperazine-1-yl)ethylamino]-2*H*-pyridazine-3-one, 500 cm³ of a 9:1 mixture of methanol and distilled water and 3.3 g of palladium on carbon catalyst consisting of 8% of Pd, 28% of C and 64% of H₂O are weighed into an apparatus of 1000 cm³ volume equipped with a reflux condenser connected to a bubbling device. 2 cm³ of hydrazine hydrate are added, drop by drop, to the reaction mixture that is then stirred at reflux temperature for 3 hours. The mixture is filtered while hot, and the catalyst is washed three times using 33 cm³ of a 1:1 mixture of methanol and dichloromethane. The combined filtrates are evaporated, and the residue is dissolved in 15 cm³ of a 8:1 mixture of ethanol and water under heating, the solution is filtered, and the filtrate is evaporated to the fourth of the original volume. After cooling, the crystals separated are stirred for further half an hour under cooling with ice water, then filtered and washed with diethyl ether. The product is dried at 60 °C over phosphorus pentoxide *in vacuo* for 3 hours.

Thus, 2.18 g (72.9 %) of the title compound are obtained. M.p.: 147-149°C.

| Analysis: for: C₁₆H₂₀FN₅O(317.37) | | | |
|---|---|---|---|
| calculated | C 64.19 %, | H 7.07 %, | N 23.39 %; |
| found | C 63.74 %, | H 7.09 %, | N 22.89 %. |

IR (KBr): 3484, 3318, 1638, 1600.
¹H-NMR (DMSO-d₆, i400): 11.98 (bs, 1H), 7.52 (d, J=2.3 Hz, 1H), 7.21 (∼t, J=7.8 Hz, 2H), 6.91 (∼d, J=8.5 Hz, 2H), 6.90 (bt, J=5.1 Hz, 1H), 6.77 (∼t, J=7.2 Hz, 1H), 5.44 (d, J=2.3 Hz, 1H), 3.14 (m, 6H), 2.56 (m, 6H).
¹³C-NMR (DMSO-d₆, i400): 162,46, 151.20, 149.47, 131.76, 129.11, 118.97, 115.52, 94.37, 55.87, 52.93, 48.30, 39.25.

### Example 4

### Preparation of 5-[2-(4-pyridin-2-ylpiperazine-1-yl)ethylamino]-2H-pyridazine-3-one

3.2 g (0.0096 moles) of 4-chloro-5-[2-(4-pyridin-2-ylpiperazine-1-yl)-ethylamino]-2*H*-pyridazine-3-one, 500 cm³ of a 9:1 mixture of methanol and distilled water and 3.2 g of palladium on carbon catalyst consisting of 8% of Pd, 28% of C and 64% of H₂O are weighed into an apparatus of 1000 cm³ volume equipped with a reflux condenser connected to a bubbling device. 1.6 cm³ of hydrazine hydrate are added, drop by drop, to the reaction mixture that is then stirred at reflux temperature for 2 hours. The mixture is filtered while hot, and the catalyst is washed three times using 33 cm³ of a 1:1 mixture of methanol and dichloromethane. The combined filtrates are evaporated, and the residue is dissolved in 30 cm³ of a 8:1 mixture of ethanol and water under heating, the solution is filtered, and the filtrate is evaporated to the fifth of the original volume. After cooling, the crystals separated are stirred for further half an hour under cooling with ice water, then filtered and washed with diethyl ether. The product is dried at 80 °C over phosphorus pentoxide *in vacuo* for 3 hours.

Thus, 2.44 g (85.0 %) of the title compound are obtained. M.p.: 150-152 °C.
IR (KBr): 3444, 1605, 1340, 1167.
¹H-NMR (CDCl₃, g200): 11.91 (bs, 1H), 8.11 (m, 1H), 7.51 (m Hz, 2H), 6.95 (b, 1H), 6.85 (m, 1H), 6.65 (m, 1H), 5.44 (s, 1H), 3.52 (m, 4H), 3.22 (m, 4H), 2.63 (m, 4H).

### Example 5

### Preparation of 5-[2-(4-pyrimidin-2-ylpiperazine-1-yl)ethylamino]-2H-pyridazine-3-one

3.4 g (0.01 moles) of 4-chloro-5-[2-(4-pyrimidin-2-ylpiperazine-1-yl)-ethylamino]-2*H*-pyridazine-3-one, 500 cm³ of a 9:1 mixture of methanol and distilled water and 3.4 g of palladium on carbon catalyst consisting of 8% of Pd, 28% of C and 64% of H₂O are weighed into an apparatus of 1000 cm³ volume equipped with a reflux condenser connected to a bubbling device. 1.7 cm³ of hydrazine hydrate are added, drop by drop, to the reaction mixture that is then stirred at reflux temperature for 1.5 hours. The mixture is filtered while hot, and the catalyst is washed three times using 33 cm³ of a 1:1 mixture of methanol and dichloromethane. The combined filtrates are evaporated, and the residue is dissolved in 28 cm³ of a 8:1 mixture of ethanol and water under heating, the solution is filtered, and the filtrate is evaporated to the fifth of the original volume. After cooling, the crystals separated are stirred for further half an hour under cooling with ice water, then filtered and washed with diethyl ether. The product is dried at 80 °C over phosphorus *pentoxide in vacuo* for 3 hours.

Thus, 2.19 g (72.5 %) of the title compound are obtained. M.p.: 199-201°C.

| Analysis: for C₁₄H₁₉N₇O (301.35) | | | |
|---|---|---|---|
| calculated | C 55.80 %, | H 6.36 %, | N 32.54 %; |
| found | C 55.71 %, | H 6.33 %, | N 32.41 %, |

IR (KBr): 3272, 3122, 1633, 1550.
¹H-NMR (DMSO-d₆, i400): 11.87 (bs, 1H), 8.35 (d, J=4.8 Hz, 2H), 7.48 (d, J=2.6 Hz, 1H), 6.85 (bt, J=5.1 Hz, 1H), 6.62 (t, J=4.7 Hz, 1H), 5.40 (∼s, 1H), 3.73 (m, 4H), 3.14 (∼q, J=6.0 Hz, 2H), 2.50 (m).
¹³C-NMR (DMSO-d₆, i400): 162.42, 161.38, 158.08, 149.44, 131.73, 110.28, 94.38, 55.85, 52.71, 43.40, 39.16.

### Example 6

### Preparation of 5-{2-[4-(3-chlorophenyl)piperazine-1-yl]ethylamino}-2H-pyridazine-3-one

A mixture of 1.96 g (0.01 moles) of 1-(3-chlorophenyl)-piperazine, 8 cm³ of dimethylformamide, 4.5 cm³ (0.014 moles) of triethylamine, 0.2 g of potassium iodide and 1.9 g (0.009 moles) of 5-(2-chloroethylamino)-2*H*-pyridazine-3-one hydrochloride is stirred at reflux temperature for 2 hours. To the reaction mixture, a solution of 3.0 g of sodium hydrogen carbonate in 40 cm³ of water is added, drop by drop. Due to the presence of the water, oil separates. The water is decanted from the oil, and 10 cm³ of dichloro-methane are added to the residue. The crystals separating under stirring are filtered. The crude product is dissolved in methanol at reflux temperature under stirring, treated with charcoal, filtered, and the filtrate is evaporated to the fifth of the original volume. The residue is stirred under cooling with ice water, and the precipitated crystals are filtered.
Thus, 1.21 g (40.5 %) of the title compound are obtained. M.p.: 187-189°C.

| Analysis: for C₁₆H₂₀ClN₅O (333.82) | | | | |
|---|---|---|---|---|
| calculated | C 57.57 %, | H 6.04 %, | Cl 10.62 %, N 20.98 %; | |
| found | C 57.09 %, | H 6.05 %, | Cl 10.38 %, | N 22.68 %. |

IR (KBr): 3410, 3277, 1624, 1599, 1240.
¹H-NMR (DMSO-d₆, i400): 11.93 (bs, 1H), 7.20 (∼t, J=8.2 Hz, 1H), 6.89 (m, 1H), 6,87 (bt, J=5.2 Hz, 1H), 6.78 (m, 1H), 5.42 (d, J=2.5 Hz, 1H), 3.18 (m, 6H), 2.54 (m, 6H).
¹³C-NMR (DMSO-d₆, i400): 162.38, 152.40, 149.42, 134.01, 131.70, 130.57, 118.17, 114.65, 113.78, 94.37,55.80,52.71, 47.74, 39.22.

### Example 7

### Preparation of 5-{2-[4-(4-fluorophenyl)piperazine-1-yl]ethylamino}-2H-pyridazine-3-one

3.51 g (0.01 moles) of 4-chloro-5-{2-[4-(4-fluorophenyl)piperazine-1-yl]ethylamino}-2*H*-pyridazine-3-one, 350 cm³ of a 9:1 mixture of methanol and distilled water, 0.44 g (0.011 moles) of sodium hydroxide and 3.5 g of palladium on carbon catalyst consisting of 8% ofPd, 28% of C and 64% of H₂O are transferred into an autoclave. The reaction mixture is stirred at room temperature under a hydrogen pressure of 10 atm for 3 hours. Then, the excess hydrogen is let out from the autoclave, the reaction mixture is heated to reflux temperature and stirred at this temperature for 5 minutes, then filtered while hot, and the catalyst is washed three times using 50 cm³ of a 1:1 mixture of methanol and dichloro methane each time. The combined filtrates are evaporated to a volume of 20 cm³, the solution obtained is stirred for half an hour under cooling with ice water, the crystals obtained are filtered and washed with 10 cm³ of cooled methanol.

Thus, 2.98 g (81.7 %) of the title compound are obtained. M.p.: 96-98°C.

| Analysis: for: C₁₆H₂₀FN₅O (317.37) | | |
|---|---|---|
| calculated | H 6.35 %, | N 22.07 %; |
| found | H 6.30 %, | N 21.64 %. |

IR (KBr) 3433, 3243, 3081, 1618, 1507, 1226.
¹H-NMR (DMSO-d₆, i400): 11.95 (bs, 1H), 7.52 (d, J=2.5 Hz, 1H), 7.02 (∼t, J=8.9 Hz, 2H), 6.94 (dd, J1=4.7 Hz, J2=9.3 Hz, 2H), 6.90 (bt, J=5.3 Hz, 1H), 5.43 (d, J=2.5 Hz, 1H), 3.15 (∼q, J=6.0 Hz, 2H), 3.08 (m, 4H), 2.56 (m, 6H).
¹³C-NMR (DMSO-d₆, i400): 162.42, 156.16 (d, J=235.4 Hz), 149.45, 148.10 (d, J=1.9 Hz), 131.72, 117.23 (J=7.6 Hz), 115.42 (d, J=21.7 Hz), 94.35, 55.82, 52.91, 49.09, 39.25.

## Claims

1. Compounds of the general Formula 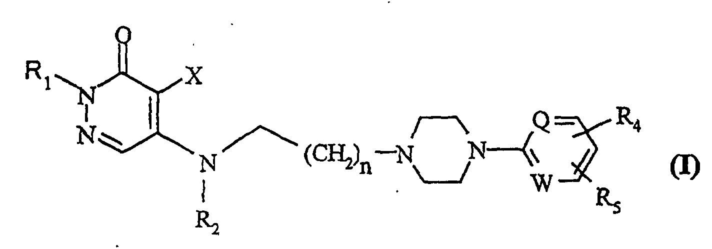 (wherein
R₁ is hydrogen or alkyl having 1-4 carbon atoms;
X is hydrogen or halogen;
R₂ is hydrogen or alkyl having 1-4 carbon atoms;
n is 1, 2 or 3;
Q and W independently from each other stand for -N= or -CH=; and
R₄ and R₅ independently from each other represent hydrogen, halogen, trifluoromethyl or alkoxy having 1-4 carbon atoms) and pharmaceutically acceptable acid addition salts thereof.

2. Compounds according to claim 1 (wherein
R₁ is hydrogen;
X is hydrogen;
n is 1;
Q and W independently from each other stand for -N= or -CH=; and
R₄ and R₅ independently from each other represent hydrogen, chlorine, fluorine, trifluoromethyl or methoxy
and pharmaceutically acceptable acid addition salts thereof.

3. 5-{2-[4-(methoxytrifluoromethylphenyl)-piperazine-1-yl]-ethylamino}-2*H*-pyridazine-3-one according to claim 1 and pharmaceutically acceptable acid addition salts thereof.

4. 5-{2-[4-(2-fluorophenyl)piperazine-1-yl]ethyl-amino}-2*H*-pyridazine-3-one according to claim 1 and pharmaceutically acceptable acid addition salts thereof.

5. 5-{2-[4-phenylpiperazine-1-yl]ethylamino}-2*H*-pyridazine-3-one according to claim 1 and pharmaceutically acceptable acid addition salts thereof.

6. 5-[2-(4-pyridin-2-ylpiperazine-1-yl)ethylamino]-2*H*-pyridazine-3-one according to claim 1 and pharmaceutically acceptable acid addition salts thereof.

7. 5-[2-(4-pyrimidin-2-ylpiperazine-1-yl)ethylamino]-2*H*-pyridazine-3-one according to claim 1 and pharmaceutically acceptable acid addition salts thereof.

8. 5-{2-[4-(3-chlorophenyl)piperazine-1-yl]ethyl-amino}-2H-pyridazine-3-one according to claim 1 and pharmaceutically acceptable acid addition salts thereof.

9. 5-{2-[4-(4-fluorophenyl)piperazine-1-yl]ethyl-amino}-2H-pyridazine-3-one according to claim 1 and pharmaceutically acceptable acid addition salts thereof.

10. Process for the preparation of compounds of the general Formula I (wherein
R₁ is hydrogen or alkyl having 1-4 carbon atoms;
X is hydrogen or halogen;
R₂ is hydrogen or alkyl having 1-4 carbon atoms;
n is 1, 2 or 3;
Q and W independently from each other stand for -N= or -CH=; and
R₄ and R₅ independently from each other represent hydrogen, halogen, trifluoromethyl or alkoxy having 1-4 carbon atoms) and pharmaceutically acceptable acid addition salts thereof which comprises
a) reacting a compound of the general Formula 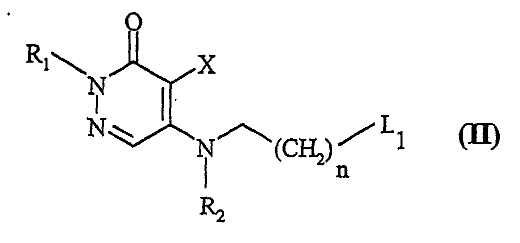 (wherein
L₁ is leaving group and R₁, R₂, X and n are as stated above) with an amine of the general Formula 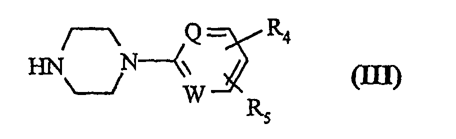 (wherein Q, W, R₄ and R₅ are as stated above); or
b) reacting a compound of the general Formula 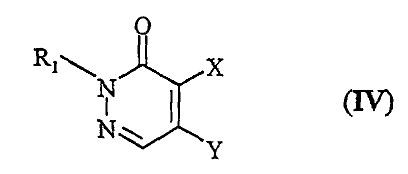 (wherein Y is halogen and R₁ and X are as stated above) with a compound of the general Formula 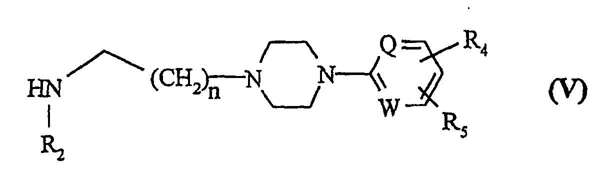 (wherein R₂, n, Q, W, R₄ and R₅ are as stated above);
and, if desired, subjecting a compound of the general Formula I, wherein X stands for halogen, to catalytic dehalogenation to obtain a compound of the general Formula I or its hydrochloride salt, wherein X is hydrogen; and/or
converting a compound of the general Formula I thus obtained into a pharmaceutically acceptable acid addition salt thereof or setting free a compound of the general Formula I from an acid addition salt.

11. Pharmaceutical composition comprising as active ingredient at least one compound of the general Formula I or a pharmaceutically acceptable acid addition salt thereof in admixture with suitable inert pharmaceutical carriers and/or auxiliary agents.

12. process for the preparation of pharmaceutical compositions which comprises admixing a compound of the general Formula I according to claim 1 or a pharmaceutically acceptable acid addition salt thereof with suitable inert pharmaceutical carriers and/or auxiliary agents.

13. Use of compounds of general Formula I according to claim 1 or a pharmaceutically acceptable acid addition salt thereof as pharmaceutical active ingredient.

14. Use of compounds of general Formula I according to claim 1 or pharmaceutically acceptable acid addition salts thereof for the preparation of medicaments having anxiolytic and cognition enhancing activities.

## Patentansprüche

1. Verbindungen der allgemeinen Formel 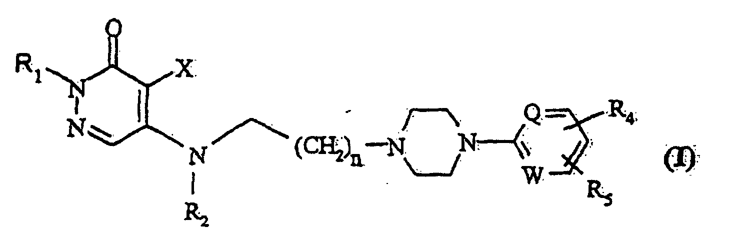 (worin
R₁ Wasserstoff oder Alkyl mit 1-4 Kohlenstoffatomen ist;
X Wasserstoff oder Halogen ist;
R₂ Wasserstoff oder Alkyl mit 1-4 Kohlenstoffatomen ist;
n 1, 2 oder 3 ist;
Q und W unabhängig voneinander für -N= oder -CH= stehen;
und
R₄ und R₅ unabhängig voneinander für Wasserstoff, Halogen, Trifluormethyl oder Alkoxy mit 1-4 Kohlenstoffatomen stehen)
und pharmazeutisch annehmbare Säureadditionssalze davon.

2. Verbindungen gemäß Anspruch 1 (worin R₁ Wasserstoff ist;
X Wasserstoff ist,
n 1 ist;
Q und W unabhängig voneinander für -N= oder -CH= stehen;
und
R₄ und R₅ unabhängig voneinander für Wasserstoff, Chlor, Fluor, Trifluormethyl oder Methoxy stehen,
und pharmazeutisch annehmbare Säureadditionssalze davon.

3. 5-{2-[4-(Methoxytrifluormethylphenyl)-piperazin-1-yl]-ethylamino}-2*H*-pyridazin-3-on gemäß Anspruch 1 und pharmazeutisch annehmbare Säureadditionssalze davon.

4. 5-{2-[4-(2-Fluorphenyl)-piperazin-1-yl]-ethylamino}-2*H*-pyridazin-3-on gemäß Anspruch 1 und pharmazeutisch annehmbare Säureadditionssalze davon.

5. 5-{2-[4-Phenylpiperazin-1-yl]ethylamino}-2*H*-pyridazin-3-on gemäß Anspruch 1 und pharmazeutisch annehmbare Säureadditionssalze davon.

6. 5-[2-(4-Pyridin-2-ylpiperazin-1-yl)-ethylamino]-2*H*-pyridazin-3-on gemäß Anspruch 1 und pharmazeutisch annehmbare Säureadditionssalze davon.

7. 5-[2-(4-Pyrimidin-2-ylpiperazin-1-yl)-ethylamino]-2*H*-pyridazin-3-on gemäß Anspruch 1 und pharmazeutisch annehmbare Säureadditionssalze davon.

8. 5-{2-[4-(3-Chlorphenyl)piperazin-1-yl]-ethylamino}-2*H*-pyridazin-3-on gemäß Anspruch 1 und pharmazeutisch annehmbare Säureadditionssalze davon.

9. 5-{2-[4-(4-Fluorphenyl)piperazin-1-yl]-ethylamino}-2*H*-pyridazin-3-on gemäß Anspruch 1 und pharmazeutisch annehmbare Säureadditionssalze davon.

10. Verfahren zur Herstellung von Verbindungen der allgemeinen Formel I (worin
R₁ Wasserstoff oder Alkyl mit 1-4 Kohlenstoffatomen ist;
X Wasserstoff oder Halogen ist;
R₂ Wasserstoff oder Alkyl mit 1-4 Kohlenstoffatomen ist;
n 1, 2 oder 3 ist;
Q und W unabhängig voneinander für -N= oder -CH= stehen;
und
R₄ und R₅ unabhängig voneinander für Wasserstoff, Halogen, Trifluormethyl oder Alkoxy mit 1-4 Kohlenstoffatomen stehen)
und pharmazeutisch annehmbare Säureadditionssalze davon) welches Folgendes umfasst
a) das Umsetzen einer Verbindung der allgemeinen Formel 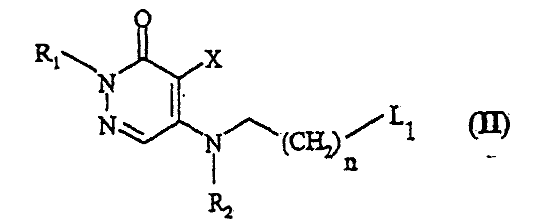 (worin
L₁ eine Abgangsgruppe ist und R₁, R₂, X und n wie oben angegeben sind) mit einem Amin der allgemeinen Formel 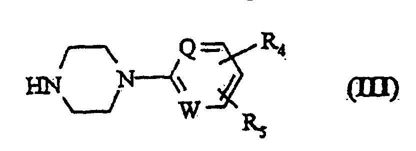 (worin Q, W, R₄ und R₅ wie oben angegeben sind); oder
b) das Umsetzen einer Verbindung der allgemeinen Formel 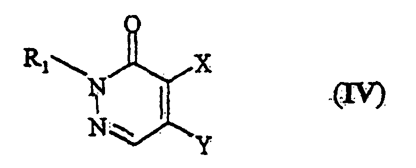 (worin Y Halogen ist und R₁ und X wie oben angegeben sind) mit einer Verbindung der allgemeinen Formel 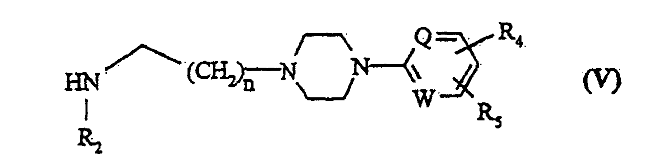 (worin R₂, n, Q, W, R₄ und R₅ wie oben angegeben sind);
und, sofern erwünscht, das Unterziehen einer Verbindung der allgemeinen Formel I, worin X für Halogen steht, einer katalytischen Dehalogenierung, um eine Verbindung der allgemeinen Formel I oder ihr Hydrochloridsalz zu erhalten, worin X Wasserstoff ist; und/oder das Umwandeln einer so erhaltenen Verbindung der allgemeinen Formel I zu einem pharmazeutisch annehmbaren Säureadditionssalz davon oder das Freisetzen einer Verbindung der allgemeinen Formel I aus einem Säureadditionssalz.

11. Pharmazeutische Zusammensetzung, umfassend als aktiven Bestandteil mindestens eine Verbindung der allgemeinen Formel I oder ein pharmazeutisch annehmbares Säureadditionssalz davon in Vermischung mit geeigneten inerten pharmazeutischen Trägem und/oder Hilfsmitteln.

12. Verfahren zur Herstellung von pharmazeutischen Zusammensetzungen, welches das Vermischen einer Verbindung der allgemeinen Formel I gemäß Anspruch 1 oder eines pharmazeutisch annehmbaren Säureadditionssalzes davon mit geeigneten inerten pharmazeutischen Trägem und/oder Hilfsmitteln umfasst.

13. Verwendung von Verbindungen der allgemeinen Formel I gemäß Anspruch 1 oder ein pharmazeutisch annehmbares Säureadditionssalz davon als pharmazeutisch aktiven Bestandteil.

14. Verwendung von Verbindungen der allgemeinen Formel I gemäß Anspruch 1 oder pharmazeutisch annehmbaren Säureadditionssalzen davon zur Herstellung von Medikamenten mit anxiolytischen oder die Kognition steigernden Aktivitäten.

## Revendications

1. Composés de formule générale : 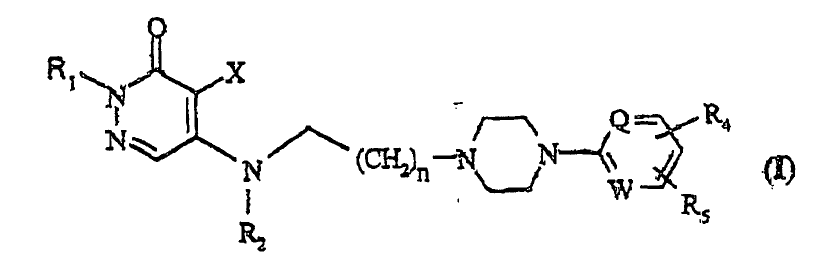 (dans laquelle
R₁ est l'hydrogène ou un alkyle ayant de 1 à 4 atomes de carbone ;
X est l'hydrogène ou un halogène ;
R₂ est l'hydrogène ou un alkyle ayant de 1 à 4 atomes de carbone ;
n vaut 1, 2 ou 3 ;
Q et W, indépendamment l'un de l'autre, désignent -N= ou -CH_; et
R₄ et R₅, indépendamment l'un de l'autre, représentent l'hydrogène, un halogène, trifluorométhyle ou un alcoxy ayant de 1 à 4 atomes de carbone)
et leurs sels d'addition d'acide pharmaceutiquement acceptables.

2. Composés selon la revendication 1, dans lesquels
R₁ est l'hydrogène ;
X est l'hydrogène ;
n vaut 1 ;
Q et W, indépendamment l'un de l'autre, désignent -N= ou -CH=; et
R₄ et R₅, indépendamment l'un de l'autre, représentent l'hydrogène, le chlore, le fluor, trifluorométhyle ou méthoxy ;
et leurs sels d'addition d'acide pharmaceutiquement acceptables.

3. 5-{2-[4-(méthoxytrifluorométhylphényl)-1-pipérazin-1-yl]éthylamino}-2H-pyridazin-3-one selon la revendication 1 et ses sels d'addition d'acide pharmaceutiquement acceptables.

4. 5-{2-[4-(2-fluorophényl)pipérazin-1-yl]éthylamino}-2H-pyridazin-3-one selon la revendication 1 et ses sels d'addition d'acide pharmaceutiquement acceptables.

5. 5-{2-[4-phénylpipérazin-1-yl]éthylamino}-2H-pyridazin-3-one selon la revendication 1 et ses sels d'addition d'acide pharmaceutiquement acceptables.

6. 5-[2-(4-pyridin-2-ylpipérazin-1-yl)éthylamino]-2H-pyridazin-3-one selon la revendication 1 et ses sels d'addition d'acide pharmaceutiquement acceptables.

7. 5-[2-(4-pyrimidin-2-ylpipérazin-1-yl)éthylamino]-2H-pyridazin-3-one selon la revendication 1 et ses sels d'addition d'acide pharmaceutiquement acceptables.

8. 5-{2-[4-(3-chlorophényl)pipérazin-1-yl]éthylamino}-2H-pyridazin-3-one selon la revendication 1 et ses sels d'addition d'acide pharmaceutiquement acceptables.

9. 5-{2-[4-(4-fluorophényl)pipérazin-1-yl]éthylamino}-2H-pyridazin-3-one selon la revendication 1 et ses sels d'addition d'acide pharmaceutiquement acceptables.

10. Procédé pour la préparation de composés de formule générale I dans laquelle
R₁ est l'hydrogène ou un alkyle ayant de 1 à 4 atomes de carbone ;
X est l'hydrogène ou un halogène ;
R₂ est l'hydrogène ou un alkyle ayant de 1 à 4 atomes de carbone ;
n vaut 1, 2 ou 3 ;
Q et W, indépendamment l'un de l'autre, désignent -N= ou -CH= ; et
R₄ et R₅, indépendamment l'un de l'autre, représentent l'hydrogène, un halogène, trifluorométhyle ou un alcoxy ayant de 1 à 4 atomes de carbone)
et de leurs sels d'addition d'acide pharmaceutiquement acceptables,
qui comprend
a) la réaction d'un composé de formule générale 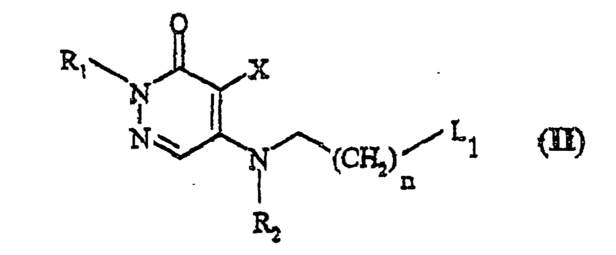 (dans laquelle L₁ est un groupe partant et R₁, R₂, X et n sont tels qu'indiqués ci-dessus)
avec une amine de formule générale 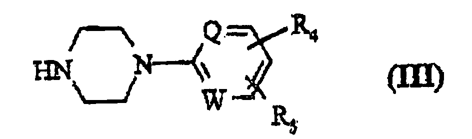 (dans laquelle Q, W, R₄ et R₅ sont tels qu'indiqués ci-dessus) ; ou
b) la réaction d'un composé de formule générale 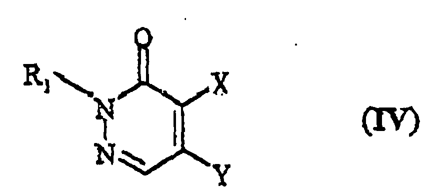 (dans laquelle Y est un halogène et R₁ et X sont tels qu'indiqués ci-dessus)
avec un composé de formule générale 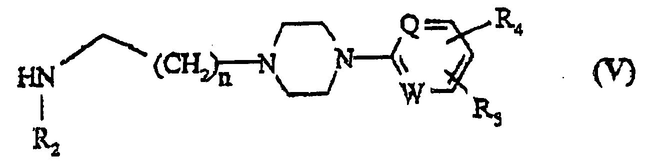 (dans laquelle R₂, n, Q, W, R₄ et R₅ sont tels qu'indiqués ci-dessus) ;
et, si on le souhaite, la soumission d'un composé de formule générale I, où X désigne un halogène, à une déshalogénation catalytique pour l'obtention d'un composé de formule générale I ou de son sel chlorhydrate, où X est l'hydrogène ; et/ou la conversion d'un composé de formule générale I ainsi obtenu en un de ses sels d'addition d'acide pharmaceutiquement acceptables ou le passage sous forme libre d'un composé de formule générale I à partir d'un sel d'addition d'acide

11. Composition pharmaceutique comprenant, à titre d'ingrédient actif, au moins un composé de formule générale I ou un de ses sels d'addition d'acide pharmaceutiquement acceptables, en mélange avec des supports pharmaceutiques inertes et/ou des agents auxiliaires convenables.

12. Procédé pour la préparation de compositions pharmaceutiques, qui comprend le mélange d'un composé de formule générale I selon la revendication 1 ou d'un de ses sels d'addition d'acide pharmaceutiquement acceptables avec des supports pharmaceutiques inertes et/ou des agents auxiliaires convenables.

13. Utilisation de composés de formule générale I selon la revendication 1 ou d'un de leurs sels d'addition d'acide pharmaceutiquement acceptables en tant qu'ingrédient pharmaceutique actif.

14. Utilisation de composés de formule générale I selon la revendication 1 ou d'un de leurs sels d'addition d'acide pharmaceutiquement acceptables pour la préparation de médicaments ayant des activités anxiolytiques et amplifiant la cognition.
